# EUROPEAN PATENT APPLICATION

(11) **EP 4 763 163 A1**
(43) Date of publication of application: **24.06.2026**
(21) Application number: 24221065.6
(22) Date of filing: 18.12.2024
(51) Int. Cl.: A61F 13/00, A61F 13/01, A61F 13/0203, A61F 13/02

(54) **AN ELECTRONIC CIRCUIT PATCH**

(71) Applicant: Mölnlycke Health Care AB, 431 21 Mölndal (SE)
(72) Inventor: JAKOBSSON, Conny, 443 38 LERUM (SE)
(74) Representative: Kransell & Wennborg KB

(57) **Abstract**

The present disclosure generally relates to an electronic circuit patch (1) for use in conjunction with a wound dressing (2) and having a lateral (x) and a longitudinal (y) extension, wherein the patch (1) comprises:
- a transfer layer (3) having a top side (3a) and a bottom side (3b), wherein at least the bottom side (3b) is adhesive;
- a support layer (4) arranged on top side (3a) of the transfer layer (3); and
- a release liner (5) releasably attached to the bottom side (3b) of the transfer layer (3), wherein the patch (1) comprises an aperture (6) extending through at least the transfer layer (3), and wherein the patch (1) comprises a first electronic circuit (7) arranged along at least a portion of the aperture (6).

The present disclosure also relates to a wound dressing assembly (9) comprising the electronic circuit patch (1) and a wound dressing (2).

## Description

### TECHNICAL FIELD

The present disclosure generally relates to an electronic circuit patch for use in conjunction with a wound dressing. The present disclosure also relates to a wound dressing assembly comprising the electronic circuit patch and a wound dressing.

### BACKGROUND

Wound infections, including surgical site infections (SSIs), represent a major challenge in post-operative and wound care management. To prevent wound infections and to secure a successful wound healing process, wound assessment is critical.

Traditionally, wound assessments have been largely qualitative, relying on visual inspections and subjective evaluations. Recent advancements have introduced the use of electrochemical sensors for quantitative wound analysis. These sensors can measure various parameters, e.g. temperature and pH, which are indicative of wound status and healing progression.

Existing sensor solutions in the field of wound care typically involve the integration of sensors and electronic circuits within wound dressings or in separate sensor sheets attached to the wound dressings. However, the integration of sensors and electronic circuits in wound dressings (or sheets) is associated with several challenges.

For example, the integration of sensors and electronic circuits in a wound dressing typically renders the dressing rigid and "bulky". This may result in discomfort for the patient, and inability of the wound dressing to conform to the contours of the skin. Accordingly, reliable wound sensing and monitoring may be impaired. Furthermore, this may result in too early detachment of the wound dressing from the skin; i.e. an impaired stay-on ability. This is particularly problematic when the wound dressing is applied onto joints or other "mobile" areas of the body.

Furthermore, precise placement of the sensors with respect to an incision (or wound) to ensure accurate readings may be a challenge. The sensors must be positioned correctly to monitor the relevant wound status parameters effectively.

Wound dressings embedded with electronic components also pose significant challenges when it comes to disposal. Such dressings are classified as hazardous waste and require careful separation of the electronic components to ensure proper disposal and recycling. This process is generally labor-intensive and costly.

Furthermore, a wound dressing embedded with sensors and other electronic components is typically complex and expensive to manufacture. Accordingly, for economical and practical reasons it is desirable that sensors and electronic components remain in contact with the skin for a prolonged time, reducing the frequency of dressing changes and ensuring continuous monitoring without interruption.

In view of this, it is desirable to provide improvements in the field of wound monitoring to secure early and reliable detection of wound infections, e.g. SSIs, while also alleviating the problems described hereinbefore.

### SUMMARY

In view of the above-mentioned problems, it is an object of the present disclosure to provide improvements in the field of wound monitoring and to provide a flexible, cost-efficient, and more sustainable means to monitor wound infections.

In a first aspect, there is provided an electronic circuit patch for use in conjunction with a wound dressing and having a lateral (x) and a longitudinal (y) extension, wherein the patch comprises:
- a transfer layer having a top side and a bottom side, wherein at least the bottom side is adhesive;
- a support layer arranged on the top side of the transfer layer; and
- a release liner releasably attached to the bottom side of the transfer layer,
wherein the patch comprises an aperture extending through at least the transfer layer, and wherein the patch comprises a first electronic circuit arranged along at least a portion of the aperture.

The present disclosure is based on the realization that an electronic circuit patch, as described hereinbefore, may be applied to the skin of a patient in a manner which mimics the application of a temporary tattoo. The transfer layer is configured to adhere to the skin of a patient. The release liner and the support layer are configured to be removed from the patch during use.

The release liner is releasably attached to the bottom side of the transfer layer and protects the transfer layer (and the patch) from contamination prior to application of the patch. Before application onto the skin, the release liner is removed.

The support layer provides structural support and integrity to the patch during application of the patch onto the skin of a patient. The support layer secures that the transfer layer can be arranged on the skin in a controlled manner. The transfer layer is typically an ultra-thin layer (micrometer or nanometer scale) and is therefore difficult to handle on its own, and extremely prone to wrinkle formation during application. Once the patch has been anchored to the skin, the support layer is configured to be removed from the patch; i.e. from the transfer layer.

The patch is provided with an aperture, which extends through at least the transfer layer. Typically, the aperture also extends through the support layer. The aperture may also extend through the release liner.

During application of the patch onto the skin, the edges of the aperture are arranged to surround, i.e. enclose, an incision (or a wound) present on the skin of a patient such that the incision (or wound) is positioned within the aperture. However, it is also conceivable that the aperture defines an area where a surgical incision is to be made on the skin (after the electronic circuit patch has been applied).

The aperture typically has a size, which substantially corresponds to the size of a wound pad of a wound dressing. During use, a wound dressing is to be arranged onto the patch such that the wound pad overlies the aperture. This way, wound liquid exuded from the incision (or the wound) is transported into the wound pad and is handled by the wound pad in an efficient manner. The size of the aperture may vary depending on the size of the wound pad and/or the wound dressing.

The patch comprises a first electronic circuit arranged along at least a portion of the aperture.

The support layer has a top side and a bottom side, wherein the first electronic circuit is arranged on the top side of the transfer layer or on the bottom side of the support layer.

In embodiments where the first electronic circuit is arranged on the bottom side of the support layer, the first electronic circuit is configured to be transferred to the top side of the transfer layer upon wetting of the support layer. In other words, in use (and after the support layer has been removed), the transfer layer carries the first electronic circuit.

Before application of a wound dressing onto the patch, at least one sensor may be connected to the first electronic circuit. For example, at least one separate sensor strip may be connected to the first electronic circuit. Data from the sensor(s) is transmitted through the first electronic circuit of the patch for further processing and external communication.

The separate sensor strip(s) may e.g. be in the form of suture sensor strip(s). Suture strips, also referred to as suture tapes are commonly used in a surgical setting to close an incision.

The provision of an aperture in the patch allows for the medical personnel to have direct access to the incision (or wound), and allows for the positioning of sensors (arranged on e.g. sensor strips) in a controlled and precise manner. The medical personnel may precisely position a sensor or a sensor strip across the incision such that a controlled sensor positioning with respect to the incision is accomplished.

The first electronic circuit is arranged along at least a portion of the aperture of the patch. This arrangement secures that an electrical connection between an electronic circuit arranged on e.g. a separate sensor strip and the first electronic circuit of the patch can be established.

It is also conceivable that sensors and electronic circuits are provided on a skin-contact surface of a wound dressing and that an electrical contact is established in a similar manner as described hereinabove with respect to the sensor strip(s). Preferably, however, the wound dressing for use in conjunction with the electronic circuit patch is void of sensors and electronic circuits.

In exemplary embodiments, the support layer is configured to be released from the top side of the transfer layer upon wetting the support layer with a wetting agent, preferably wherein the wetting agent is selected from water (e.g. sterile water), saline, and an antiseptic solution.

Hence, the mode of application of the patch resembles that of a temporary tattoo. Such an application mode is user-friendly and intuitive for healthcare professionals.

The support layer may e.g. be configured to be released from the top side (upon wetting) by the incorporation of a water-soluble coating in the patch.

Accordingly, in exemplary embodiments, the electronic patch may further comprise a water-soluble coating arranged on the bottom side of the support layer.

When the support layer is wetted with a wetting agent, the water-soluble coating on the bottom side of the support layer dissolves. This dissolution weakens the bond between the support layer and the transfer layer, allowing the transfer layer to separate from the support layer. The transfer layer is pressed against the skin during this process, ensuring that it adheres securely to the patient's skin as the support layer is removed. This way, the transfer layer may conform closely to the contours of the skin. After the support layer has been wetted, and the water-soluble coating has dissolved, the support layer can easily be peeled away from the patch.

In embodiments where the first electronic circuit is arranged on the bottom side of the support layer, the first electronic circuit is arranged on the water-soluble coating. In other words, when the water-soluble coating dissolves (upon wetting of the support layer), the first electronic circuit will be transferred to the underlying transfer layer.

In alternative embodiments, the support layer may be removed from the top side of the transfer layer in a different manner. For example, a relatively weak adhesion between the support layer and the top side of the transfer layer may allow the support layer to be peeled off without wetting. Accordingly, the electronic circuit patch may be regarded a "non-water-based" temporary tattoo. In such embodiments, the first electronic circuit may be arranged on the top side of the transfer layer.

In some embodiments, one or a plurality of sensors may be arranged on the first electronic circuit. Such sensors may e.g. be reference sensors configured to monitor parameters connected to the wound status in an area remote from the incision or wound.

In exemplary embodiments, the top side of the transfer layer is also adhesive.

Accordingly, the transfer layer may be a double-sided adhesive transfer layer. The adhesive on the top side of the transfer layer secures that the support layer remains attached to the transfer layer before application onto the skin, e.g. before wetting. The adhesive on the bottom side of the transfer layer secures that the release liner remains attached to the transfer layer prior to use. Furthermore, the adhesive bottom side secures a firm adhesion to the skin after the release liner has been removed.

In embodiments where a water-soluble coating is arranged on the bottom side of the support layer, at least a portion of the water-soluble coating may be transferred to the top side of the transfer layer. This may reduce or eliminate the adhesiveness of the top side of the transfer layer. The first electronic circuit, if/when transferred from the bottom side of the support layer to the top side of the transfer layer, may be at least partially covered with the dissolved water-soluble coating.

In exemplary embodiments, the transfer layer may have a thickness in the range of from 0.2 to 20 µm, preferably from 0.4 to 10 µm, more preferably from 1.0 to 6.0 µm.

Accordingly, the transfer layer is an ultrathin, and flexible layer, which is extremely conformable to the skin. The transfer layer adheres closely to the natural contour of the skin of a patient and is experienced by the patient as a "natural extension" of the skin.

In exemplary embodiments, the patch is divided into three separate portions along the lateral (x) extension of the patch: a first lateral edge portion, a central portion, and a second lateral edge portion, wherein the aperture may be arranged in the central portion of the patch.

As mentioned hereinbefore, the aperture extends through at least the transfer layer. Typically, the aperture also extends through the support layer. This facilitates the positioning of the patch onto an incision (or wound) on the skin of a patient.

During application, the medical personnel may grasp the patch at the lateral edge portions and position the aperture with respect to an incision or wound. The lateral edge portions of the patch provide anchoring points for the patch onto the skin. The centrally arranged aperture facilitates the subsequent arrangement of a sensor strip on the skin and/or incision, and a wound pad of a medical dressing thereon.

In exemplary embodiments, the aperture may have a surface area of from 5 to 100 cm², preferably from 10 to 50 cm².

Hence, the surface area of the aperture corresponds to the typical size (or surface area) of a wound pad used in a wound dressing. Since wound dressings come in a variety of sizes, the size of the wound pad, and thus the aperture of the patch, may vary.

The wound pad is positioned on the aperture such that the wound pad covers the aperture, and effectively manages wound exudate without leaving gaps or "overextending" beyond the aperture. The wound pad may thus directly engage with the wound site and handle the exudate.

In exemplary embodiments, the aperture is defined by a first lateral edge, a second lateral edge, a first longitudinal edge and a second longitudinal edge; the first and second lateral edges extending in the longitudinal (y) direction of the patch; the first and second longitudinal (y) edges extending in the lateral (x) direction of the patch, and wherein the first electronic circuit comprises a first branch extending adjacent to and along the first longitudinal edge of the aperture, and a second branch extending adjacent to and along the second longitudinal (y) edge of the aperture.

The longitudinal edges typically run parallel to the incision enclosed by the aperture of the patch. By arranging the circuit branches adjacent to and along the longitudinal edges of the aperture, the connection with sensors provided on e.g. separate sensor strips, such as suture sensor strips, is greatly facilitated. This arrangement also facilitates the provision of reference sensors in an area remote from the incision or wound site.

In exemplary embodiments, the first and second branches of the first electronic circuit may extend uninterrupted to an electronic connector arranged in the second lateral edge portion of the patch.

The electronic connector serves as an interface between the first electronic circuit (and sensors connected to the circuit), and an external processing device.

The processing device may be attached to the electronic connector and is configured to receive and evaluate data from sensors connected to the first electronic circuit.

The electronic connector is arranged in the second lateral edge portion of the electronic circuit patch. This allows for the processing device to be coupled to the electronic circuit patch in a location remote from the incision (or wound), and outside the peripheral edges of a wound dressing, which is arranged on the central portion of the patch.

This "remote" location facilitates battery changes, data retrieval, and other maintenance activities without disturbing the wound (or incision) site or compromising the integrity of the patch or the wound dressing. By having the electrical connector (and thus also the processing device) positioned away from the wound site and the wound dressing, there is also less "bulk" and discomfort for the patient.

Furthermore, the wound dressing may be detached from the electronic circuit patch, when saturated, i.e. when the wound dressing has reached its full capacity, and replaced with a new one, while keeping the electronic circuit patch (and sensor suture strips) attached to the skin. The wear time of the patch (and the components responsible for wound monitoring) may thus be significantly prolonged compared to e.g. conventional wound dressings comprising integrated sensors.

According to another aspect, there is provided a wound dressing assembly comprising the electronic circuit patch as described hereinbefore, and a wound dressing, wherein the wound dressing comprises a wound pad, and wherein the wound pad is arranged on top of the aperture of the patch during use.

The wound dressing is defined by peripheral edges, and wherein at least the electronic connector may be arranged in an area outside the peripheral edges of the wound dressing during use.

As mentioned hereinbefore, this is beneficial to avoid interference with the wound site. Furthermore, it allows for connection with an external processing device without interfering with the wound dressing or the monitoring of the wound characteristics.

In exemplary embodiments, the wound pad is arranged in a central portion of the dressing, and wherein the wound dressing may further comprise an adhesive border portion surrounding the central portion; the adhesive border portion being arranged to extend beyond the first and second lateral edges and the first and second longitudinal edges of the aperture during use.

Accordingly, the wound pad aligns with the aperture of the patch and is in direct contact with the wound site, while the border portion anchors the wound dressing to the portions of the patch surrounding the aperture, and to the skin of a patient (depending on the size of the border portion). The adhesive border portion seals the wound dressing to the patch (and skin) and prevents leakage of wound exudate.

In exemplary embodiments, the wound dressing assembly may further comprise at least one sensor strip, wherein the sensor strip comprises a second electronic circuit extending in a length direction of the sensor strip and at least one sensor connected to the electronic circuit, preferably wherein the sensor strip is a suture sensor strip.

The sensor strip(s) may be arranged on the skin of a patient such that the second electronic circuit intersects with the first electronic circuit, e.g. the first and second branches of the first electronic circuit. This way, an electrical contact is established between the first and second electronic circuits.

The at least one sensor strip may be positioned on the skin such that it crosses over the incision (or wound). The skin, and the incision are made available by the aperture in the transfer layer. The at least one sensor strip is arranged across the first and second longitudinal edges of the aperture. In other words, the at least one sensor strip is arranged perpendicularly to the incision (and perpendicularly to the first and second branches).

The sensor strip has a top side and a bottom side. Typically, the bottom side of the sensor strip is adhesive.

The second electronic circuit and the at least one sensor are typically arranged on the bottom side of the sensor strip. Hence, the sensor(s) may be provided in direct contact with the incision (or wound).

The sensor(s) are typically arranged in an area of the sensor strip which is to be arranged above, or at least in the vicinity of, the incision (or wound). Typically, the at least one sensor is arranged centrally along the length of the sensor strip.

The at least one sensor of the sensor strip(s) may monitor parameters indicative of wound status or wound infection, e.g. temperature, pH, moisture and/or biochemical markers related to the status of the wound.

As mentioned hereinbefore, the data collected by the sensor(s) is transferred by means of the first and second electronic circuits to the electrical connector and a processing device attached thereto.

Typically, a plurality of sensor strips is utilized. This way, sensor measurements may be accomplished along the entire incision (or wound). Preferably, the sensor strips are suture sensor strips. Hence, the sensor strips serve the dual purpose of assisting with wound or incision closure while also securing monitoring of the wound status.

The sensor strips allow for precise and controlled alignment of the sensors with respect to an incision (or wound) such that accurate sensor readings and an improved monitoring of wound status is accomplished.

In exemplary embodiments, the wound dressing assembly may further comprise a wetting agent.

The wetting agent may be sterile water, saline, or an antiseptic solution. The choice of wetting agent depends on the clinical context, such as the need to disinfect the skin prior to application.

Preferably, the wetting agent is an antiseptic solution. Using an antiseptic solution (e.g. a chlorhexidine solution) as the wetting agent is beneficial as it also pre-cleans and disinfects the incision site.

In exemplary embodiments, the wound dressing assembly may further comprise a separate processing device configured for connection with the electronic connector, preferably wherein the processing device is arranged in a conformable pad.

The integration of the processing device within a conformable pad ensures that the processing device (and the entire wound dressing assembly) is formable, flexible, and user-friendly. The processing device can be easily attached to the electrical connector of the electronic circuit patch without interfering with the wound site, and the wound dressing. This significantly simplifies the handling for medical personnel. Furthermore, the wound dressing assembly is associated with "softer" (and less bulky) components, which is convenient for the patient. All components of the wound dressing assembly can thus conform and adapt to the movement of a patient.

In exemplary embodiments, the wound dressing is void of sensors and electronic circuits.

Hence, when the wound dressing is saturated, it may easily be replaced with a new wound dressing. This way, the costs associated with several dressing changes (as is the case when the dressing is embedded with sensors and/or electronic components) can be significantly reduced. Furthermore, the major parts of the used materials can be handled as regular medical waste, which significantly reduces the burden on hazardous waste management. The electronic circuit patch and sensors connected thereto remain on the skin and provide continuous (non-interrupted) real-time data and monitoring of the wound or incision.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1a schematically illustrates a split view of an electronic circuit patch according to an exemplary embodiment of the present disclosure.
Figure 1b schematically illustrates a top-view of an electronic circuit patch, as seen from the top side of the transfer layer. In figure 1b, the support layer has been removed from the patch.
Figures 2a-h schematically illustrate the steps of applying the electronic circuit patch onto the skin of patient, as well as additional components comprised in a wound dressing assembly according to an exemplary embodiment of the present disclosure.
Figure 3 schematically illustrates a partially split view of a wound dressing for use in conjunction with the electronic circuit patch or forming part of the wound dressing assembly according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

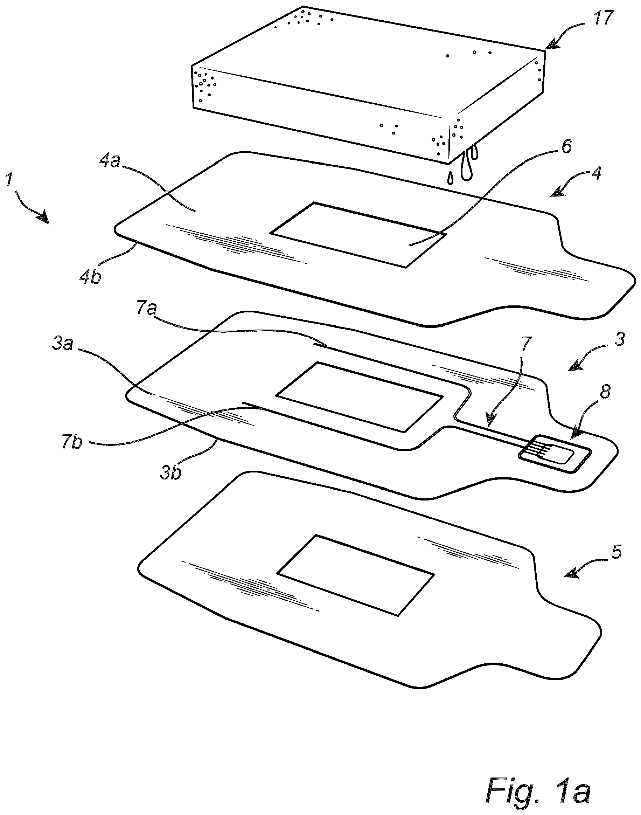

The electronic circuit patch will now be explained with reference to figures 1a-1b. The electronic circuit patch 1 has a lateral (x) and a longitudinal (y) extension, wherein the patch 1 comprises:
- a transfer layer 3 having a top side 3a and a bottom side 3b, wherein at least the bottom side 3b is adhesive;
- a support layer 4 arranged on the top side 3a of the transfer layer 3; and
- a release liner 5 releasably attached to the bottom side 3b of the transfer layer 3, wherein the patch 1 comprises an aperture 6 extending through at least the transfer layer 3, and wherein the patch 1 comprises a first electronic circuit 7 arranged along at least a portion of the aperture 6.

As used herein, the term "electronic circuit patch" means a flexible and conformable patch which comprises at least a transfer layer, a support layer, and a release liner. The patch comprises a first electronic circuit. The first electronic circuit may be arranged on the top side of the transfer layer or on a bottom side of the support layer. The transfer layer of the patch is configured to be transferred from the patch and adhere to the skin during use. The electronic circuit patch functions similarly to a temporary tattoo. During use, both the support layer and the release liner are configured to be removed from the patch.

The "transfer layer" is configured to adhere to the skin of a patient. At least the bottom side 3b of the transfer layer is adhesive. The bottom side 3b adheres to the skin of a patient during use. The top side 3a of the transfer layer may also be adhesive. The top side 3a adheres to the support layer 4 of the patch.

The transfer layer is an extremely thin and flexible layer. Typically, the thickness of the transfer layer is in the range of from 0.2 to 20 µm, preferably from 0.4 to 10 µm, more preferably from 1.0 to 6.0 µm.

The transfer layer is typically transparent, or at least substantially transparent. Hence, light is allowed to pass through the transfer layer such that the first electronic circuit can be visibly observable during use.

The bottom side of the transfer layer may comprise an adhesive coating, e.g. continuous or a discontinuous coating. The adhesive may e.g. be a pressure-sensitive adhesive. The top side of the transfer layer may also comprise an adhesive coating. The invention is by no means limited to a specific adhesive. The adhesive used on the top side may be the same as the adhesive used on the bottom side. Alternatively, the adhesive used on the top side is different from the adhesive used on the bottom side.

The transfer layer may comprise any material which is skin-friendly, and capable of forming a thin film, and which may withstand mechanical stress without degrading or tearing. For example, the transfer layer may be a polymeric film, e.g. a polyurethane film, a silicone film, or a cellulose-based film, e.g. a film comprising cellulose acetate or ethylcellulose.

The support layer 4 is configured to be removed from the patch after application onto the skin. The support layer 4 provides structural rigidity to the patch and facilitates the application of the patch onto the skin.

The support layer 4 may be configured to be released from the top side 3a of the transfer layer 3 upon wetting the support layer with a wetting agent, preferably wherein the wetting agent is selected from water, saline, and an antiseptic solution.

When the support layer 4 is wetted, e.g. with sterile water or an antiseptic solution, it may easily be peeled off from the transfer layer.

As mentioned hereinbefore, in some embodiments, the support layer 4 may be configured to be removed from the top side of the transfer layer without wetting.

In the context of the present disclosure, the "water-based tattoo transfer" is generally preferred.

The support layer typically comprises paper. Alternatively, the support layer may comprise a mixture of paper and synthetic polymers.

As illustrated in figure 1a, the support layer 4 has a top side 4a and a bottom side 4b. The electronic patch 1 may further comprise a water-soluble coating arranged on the bottom side 4b of the support layer 4.

The water-soluble coating dissolves or weakens upon contact with a liquid. Accordingly, the adhesion between the support layer 4 and the transfer layer 3 is reduced such that the support layer may easily be peeled off, and such that the transfer layer adheres to the skin.

Furthermore, in embodiments where the first electronic circuit is provided, e.g. printed on the bottom side 4a of the support layer 4, the first electronic circuit will be transferred to the underlying transfer layer upon dissolution of the water-soluble coating.

The water-soluble coating may comprise a starch-based polymer, e.g starch-dextrin, carboxymethyl cellulose (CMC), or hydroxypropyl methylcellulose (HPMC).

The release liner 5 may comprise any suitable release liner material known in the art, e.g. polyethylene, polyester, polypropylene, silicone coated paper, and combinations thereof.

In figure 1a, the first electronic circuit 7 is illustrated as being arranged on the top side 3a of the transfer layer 3. It is, however, equally conceivable, and sometimes more preferred, that the first electronic circuit is arranged on the bottom side 4b of the support layer 4.

The first electronic circuit 7 may be arranged on the top side of the the transfer layer or on the bottom side of the support layer by e.g. thin-film deposition or printing. For example, the first electronic circuit may be arranged on the transfer layer or support layer by screen printing, inkjet printing, vacuum deposition or sputtering. A conductive ink may be used for printing.

It is also conceivable that the first electronic circuit is arranged on the bottom side 3b of the transfer layer. For example, the first electronic circuit may be arranged on the bottom side 3b of the transfer layer prior to applying an adhesive coating onto the bottom side.

The transfer layer 3 and the support layer 4 are typically co-extensive in length and in width. In other words, the surface area of the transfer layer 3 corresponds to the surface area of the support layer 4.

Typically, the surface area of the release liner 5 corresponds to the surface area of the transfer layer 3 and the support layer, as e.g. illustrated in figure 1a.

The electronic circuit patch 1 comprises an aperture 6, which extends through at least the transfer layer 3, and typically also through the support layer 4. The aperture 6 may also extend through the release liner 5, as illustrated in e.g. figure 1a.

In figure 1a, the aperture 6 is rectangular. However, the aperture is not limited to a rectangular shape, but any shape is conceivable, such as oval, square, round etc. Typically, however, the aperture has a substantially rectangular shape.

The aperture may have a surface area of from 5 to 100 cm², preferably from 10 to 50 cm². The size, i.e. surface area, of the aperture may vary depending on the size of the dressing, the size of the wound pad, and the characteristics of the wound or incision to be treated.

The maximum lateral (x) extension, i.e. the length, of the aperture 6 may be from 5 to 35 cm, e.g. from 8 to 40 cm.

The maximum longitudinal (y) extension, i.e. the width, of the aperture may be from 2 to 20 cm, e.g. from 2.5 to 10 cm.

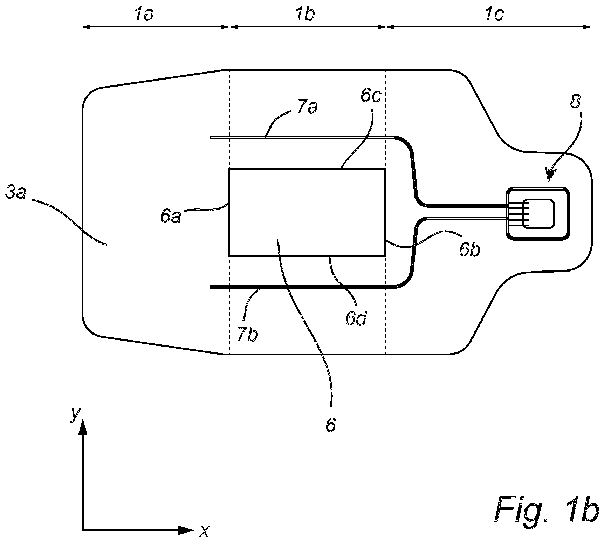

As best illustrated in figure 1b, the electronic circuit patch 1 may be divided into three separate portions along the lateral (x) extension of the patch 1: a first lateral edge portion 1a, a central portion 1b, and a second lateral edge portion 1c, wherein the aperture 6 is arranged in the central portion 1b of the patch 1.

This arrangement facilitates the application of the patch onto the skin of a patient, as well as the arrangement of a wound pad on top of the aperture. It also yields a more cohesive and visually appealing impression.

In figure 1b, the support layer has been removed from the patch to clearly illustrate the separate portions of the patch.

In e.g. figure 1b, the second lateral edge portion 1c has a tapering configuration. The second lateral edge portion 1c may taper towards a peripheral edge of the patch. The invention is by no means limited to this construction. However, this construction may be beneficial to facilitate the application of an external processing device onto the electronic connector arranged in this part of the patch. It may also be beneficial to minimize the usage of patch materials.

As best illustrated in figure 1b, the aperture 6 is defined by a first lateral edge 6a, a second lateral edge 6b, a first longitudinal edge 6c and a second longitudinal edge 6d; the first 6a and second 6b lateral edges extending in the longitudinal (y) direction of the patch 1; the first 6c and second 6d longitudinal (y) edges extending in the lateral (x) direction of the patch 1, and wherein the first electronic circuit 7 comprises a first branch 7a extending adjacent to and along the first longitudinal edge 6c of the aperture 6, and a second branch 7b extending adjacent to and along the second longitudinal (y) edge 6d of the aperture 6.

The first 7a and second 7b branches of the first electronic circuit are preferably arranged adjacent to the aperture. This is to facilitate connection with e.g. a sensor strip and establishing electrical connection to the sensor(s) of such sensor strip(s).

The first branch 7a extends in parallel and adjacent the first longitudinal edge 6c of the aperture 6. For example, the distance between the first branch 7a and the first longitudinal edge 6c may be from 2 to 15 mm, e.g. from 3 to 10 mm.

The second branch 7b extends in parallel and adjacent the second longitudinal edge 6d of the aperture 6. For example, the distance between the second branch 7b and the second longitudinal edge 6d may be from 2 to 15 mm, e.g. from 3 to 10 mm.

Typically, the first 7a and second 7b branches of the first electronic circuit 7 extend uninterrupted to an electronic connector 8 arranged in the second lateral edge portion 1c of the patch 1.

As used herein, the term "electronic connector" means a conductive interface component configured to establish an electrical connection with an external processing device.

The electrical connector 8 may be connected to at least one sensor (either integrated in the first electronic circuit 7 or electrically connected thereto). The first electronic circuit 7 transmits signals and power between the sensor(s) and an external processing device (connected to the electrical connector 8).

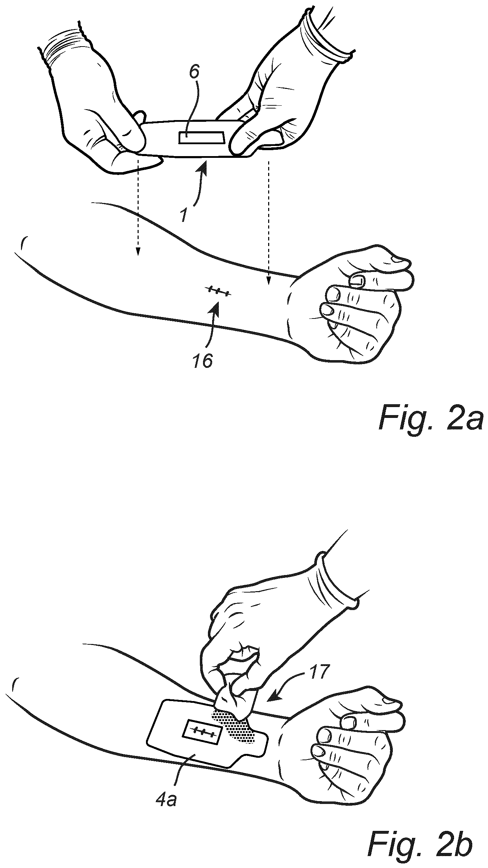

According to another aspect, there is provided a wound dressing assembly 9 comprising the electronic circuit patch 1 as described hereinbefore, and a wound dressing 2, wherein the wound dressing 2 comprises a wound pad 10, and wherein the wound pad 10 is arranged on top of the aperture 6 of the patch 1 during use (see figures 2a-h).

Figures 2a-h illustrate the application of the electronic circuit patch 1, and other components associated with a wound dressing assembly according to an exemplary embodiment of the present disclosure.

As illustrated in figure 2a, a caregiver may grasp the electronic circuit patch 1 at the first and second lateral edges of the electronic circuit patch 1, and subsequently apply the patch onto the skin such that the aperture 6 is arranged to enclose an incision 16 present on the skin of a patient.

Prior to positioning the patch 1 onto the skin, the release liner is removed (not shown).

As mentioned hereinbefore, it is also conceivable that the patch 1 is positioned onto intact skin, and that a surgical incise is subsequently provided in the area of the skin where the aperture is positioned.

After the electronic circuit patch 1 has been applied to the skin, the caregiver may wet the support layer 4, as illustrated in figure 2b. This may be accomplished by using a suitable wetting applicator 17, e.g. a cloth, gauze, foam, napkin, or similar, soaked with a wetting agent. The applicator typically wets the entire top side 4a of the support layer. The wetting agent may be sterile water, saline, or an antiseptic solution (e.g. a chlorhexidine solution). By using an antiseptic solution as the wetting agent, the skin is simultaneously disinfected, which is beneficial in a surgical setting.

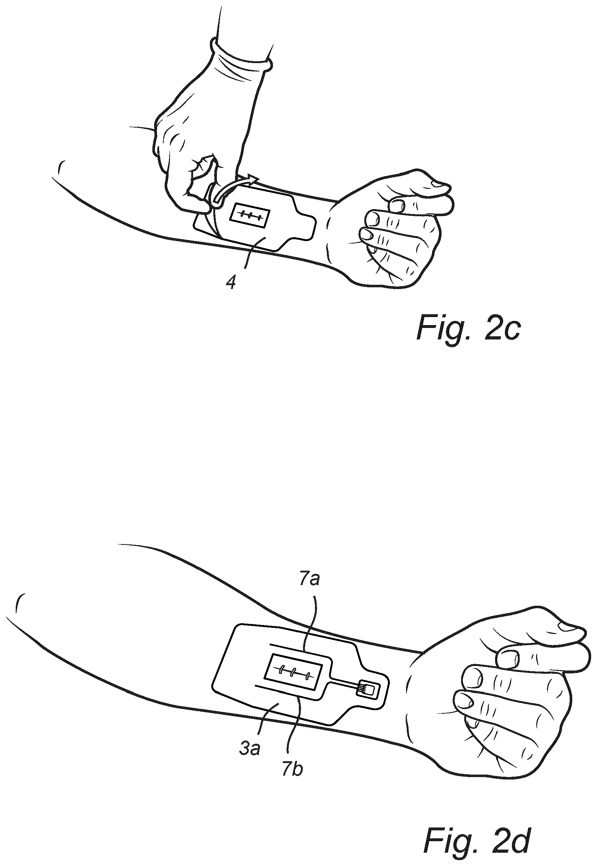

When the support layer 4 has been wetted, the caregiver may easily peel off the support layer 4 from the patch 1, as illustrated in figure 2c.

Figure 2d illustrates the transfer layer 3 of the patch (seen from the top side 3a) arranged on the skin of a patient. The first 7a and second 7b branches of the first electronic circuit 7 are typically arranged parallel to the first 6c and second 6d longitudinal edges of the aperture. Since the incision 16 is typically straight, the first 7a and second 6b branches are typically arrange parallel to the incision. As can be seen, the transfer layer 3 now forms the top layer of the patch 1 (after removal of the support layer 4). Hence, during subsequent assembly with at least one sensor strip, and a wound dressing, only the transfer layer of the patch remains on the skin.

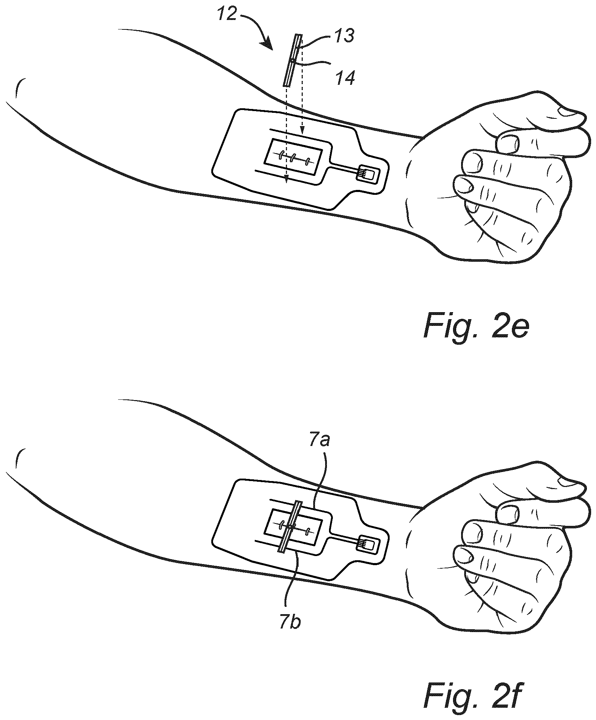

The wound dressing assembly of the present disclosure may further comprise at least one sensor strip 12. As illustrated in figure 2e, the sensor strip 12 may be a suture sensor strip.

As used herein, the term "suture sensor strip" means a strip or band configured to be applied over an incision or wound and assist in closing, stabilizing, and protecting the incision/wound. The suture sensor strip may also be referred to as a "suture sensor tape". Typically, the sensor strip has a top side and a bottom side, wherein at least the bottom side is adhesive.

The sensor strip 12 comprises a second electronic circuit 13 extending in a length direction of the sensor strip 12 and at least one sensor 14 connected to the second electronic circuit 13.

As illustrated in figures 2e and 2f, the sensor strip 12 is arranged across the aperture (and the incision) such that the strip (and the second electronic circuit 13) intersect with the first 7a and second 7b branches of the first electronic circuit. This way, an electrical connection is provided.

The at least one sensor 14 of the sensor strip 12 is preferably arranged centrally along the length of the strip (as illustrated in figures 2e-f). This way, the sensor may be positioned above and in direct contact with the incision 16 (or wound). It is also conceivable that a plurality of sensors is arranged along the length of the sensor strip 12.

The sensor strip 12 has a top side and a bottom side, wherein the second circuit 13 and the at least one sensor 14 are typically arranged on the bottom side. In figures 2e-g, the top side of the sensor strip is shown. The sensor strip 12 is, in these figures, illustrated as being transparent to more clearly illustrate the circuit 13 and the sensor 14.

The at least one sensor may be configured to monitor at least one parameter indicative of infection. Accordingly, accurate and timely detection of wound infections, e.g. SSIs can be accomplished. The sensor readings help medical personnel to quickly identify trends or anomalies. For example, a gradual increase in temperature may indicate wound infection, which may prompt timely intervention.

The at least one sensor may be a temperature sensor, pH sensor, humidity sensor, and/or a sensor configured to measure dissolved molecules (e.g. oxygen, glucose, ions) in the wound. Preferably, the at least one sensor is a temperature sensor or a pH sensor.

Typically, a plurality of sensor strips 12; i.e. suture sensor strips is used. For ease of illustration, only one sensor strip 12 is illustrated in figures 2e-f.

The size of the sensor strip 12 may vary depending on the size of the aperture of the patch, as well as the size of the wound dressing and the wound pad. Typically, the maximum lateral (x) extension; i.e. the length of the suture sensor strip is larger than the maximum longitudinal (y) extension of the aperture of the patch. This is to enable electrical connection between the second electronic circuit 13 and the first electronic circuit of the patch.

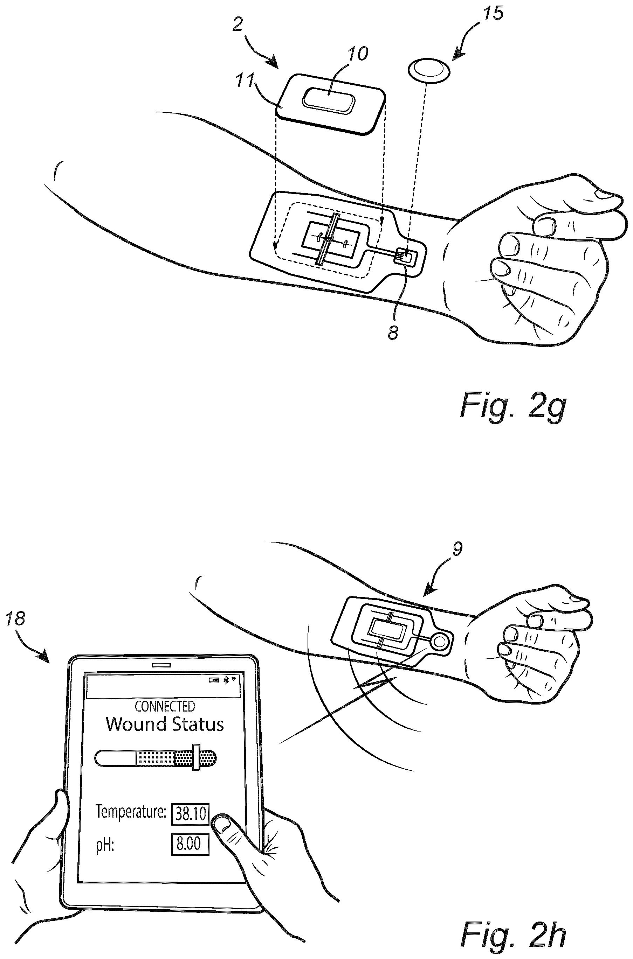

The wound dressing assembly of the present disclosure further comprises a wound dressing 2 comprising a wound pad 10. The wound pad 10 is arranged on top of the aperture 6 of the patch 1 during use, as illustrated in figure 2g.

As mentioned hereinbefore, the size of the wound pad 10 typically corresponds to the size of the aperture 6 of the patch 1. Accordingly, the caregiver may align the wound pad 10 with the aperture 6 of the patch 1 during application, as illustrated in figure 2g.

The wound dressing 2 is defined by a peripheral edges. As illustrated in figure 2g (and figure 2h), at least the electronic connector 8 is arranged in an area outside the peripheral edges of the wound dressing 2 during use.

Typically, the wound pad 10 is arranged in a central portion of the dressing, and wherein the wound dressing 2 further comprises an adhesive border portion 11 surrounding the central portion; the adhesive border portion 11 being arranged to extend beyond the first 6a and second 6b lateral edges and the first 6c and second 6d longitudinal edges of the aperture 6 during use (see e.g. figure 2g).

The wound dressing 2 may thus be referred to as a "border dressing". The adhesive border portion 11 is arranged onto the patch in the area surrounding the aperture (see dotted lines in figure 2g), whereas the wound pad 10 is arranged to cover the aperture.

The wound dressing assembly may further comprise a separate processing device configured for connection with the electronic connector 8, preferably wherein the processing device is arranged in a conformable pad 15 (see e.g. figure 2g).

As used herein, the term "processing device" means a component comprising a power source, a processing unit, and means to communicate data with an electronic device, designed to receive, analyze, and transmit data from the sensors.

The conformable pad 15 may be formed from conventional dressing materials or be formed from injection-molded foam, plastic, or other polymeric materials. Typically, the conformable pad 15 comprises a formable and flexible material.

The conformable pad 15 comprising the processing device may be attached to the electrical connector 8 by adhesive or mechanical means. The conformable pad is attached to the electrical connector such that an electrical connection is provided.

The conformable pad 15 may have the same general construction as a small wound dressing. The conformable pad 15 may e.g. comprise a central portion and a border portion surrounding the central portion. The central portion of the pad 15 may comprise the processing device. The border portion may be adhesive. Hence, the adhesive border portion of the pad 15 may be attached to the skin of a patient (and/or onto the patch) during use.

In the context of the present disclosure, the wound dressing is preferably a wound dressing adapted for post-operative incisions. Such a dressing is typically a so called border dressing.

As mentioned hereinbefore, a border dressing comprises a wound pad arranged in a central portion of the wound dressing, and an adhesive border portion surrounding the central portion. The adhesive border portion may be formed from a backing layer (i.e. top layer) configured to extend beyond the edges of the wound pad. At least the areas of the backing layer that extend beyond the edges of the wound pad may be provided with an adhesive coating. The wound pad may be arranged to contact the skin or it may be covered with an adhesive skin contact layer.

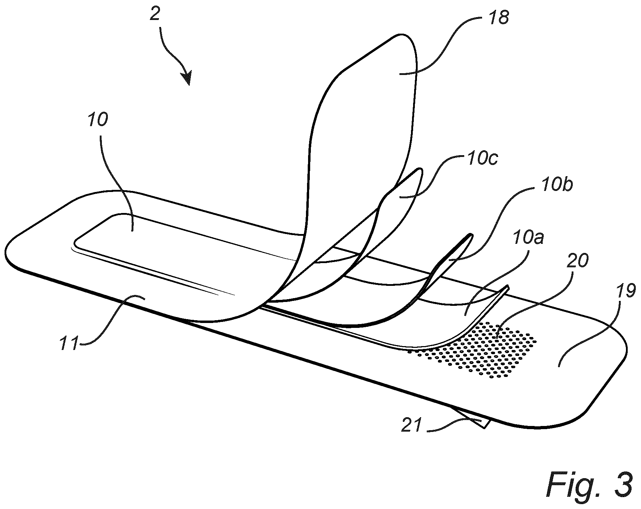

An exemplary wound dressing 2 suitable for use in the wound dressing assembly is illustrated in figure 3.

The wound dressing 2 may comprise a backing layer 18, an adhesive skin-contact layer 19, and a wound pad 10 arranged between the backing layer 18 and the adhesive skin-contact layer 19; the wound pad 10 being defined by pad edges, wherein the backing layer 18 and the adhesive skin-contact layer 19 are arranged to extend beyond the pad edges to form a border portion 11 surrounding the wound pad 10.

The "backing layer" is the top layer; i.e. the outermost layer of the wound dressing. The backing layer is a continuous layer and protects the layers of the dressing from entry of potential contaminants. The backing layer typically comprises a polymeric film, e.g. a polyurethane film. The thickness of the backing layer may be in the range of from 15 to 50 µm, preferably from 20 to 40 µm.

The "adhesive skin-contact layer" of the wound dressing is configured to detachably adhere the dressing to a dermal surface and/or the electronic circuit patch.

The adhesive skin-contact layer 19 may comprise a polymeric film, e.g. a polyurethane film, and a silicone-based adhesive coating; the silicone-based adhesive coating being arranged to contact the skin of a wearer during use.

As can be seen in figure 3, the adhesive skin-contact layer 19 may comprise a plurality of perforations 20 in the area underlying the wound pad 10, but is void of perforations in the area forming the border portion 11.

This construction is beneficial to improve the adherence of the dressing onto the skin and the electronic circuit patch; i.e. to enhance the wear time of the wound dressing. It is also beneficial to prevent leakage of exudate from the wound pad towards the border portion.

In the context of the present disclosure, the "wound pad" may comprise one or a plurality of pad-forming layers. Typically, the wound pad comprises more than one layer.

The wound pad may e.g. comprise an absorbent polyurethane foam layer.

The wound pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The wound pad may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the wound dressing.

For example, the wound pad illustrated in figure 3 may comprise a first absorbent layer 10a, a liquid distribution layer 10b and a second absorbent layer 10c. Typically, the liquid distribution layer 10b is arranged between the first 10a and the second 10c absorbent layers, wherein the first absorbent layer 10a is the lowermost layer of the wound pad.

The first absorbent layer 10a may comprise a foam. Suitable foam materials for use in the first absorbent layer 10a include, but are not limited to polyurethane foams.

The second absorbent layer 10c may be a superabsorbent layer. Accordingly, the second absorbent layer may comprise superabsorbent polymers (SAP) or superabsorbent fibers (SAF).

The liquid distribution layer 10b may comprise any material having the ability to distribute the exudate in an efficient manner. For example, the liquid distribution layer 10b may comprise a nonwoven material. A nonwoven imparts an appropriately balanced rigidity to the layer and to the dressing as such. It may also efficiently distribute and spread liquid absorbed by the first absorbent layer 10a such that it can be evaporated through the backing layer 18 over a large surface. For example, the nonwoven may comprise viscose, polyester or blends thereof.

The layers can be joined by adhesion, lamination, using e.g. pressure and heat.

The wound pad 10 may comprise additional layers, such as liquid transport layers, various combinations of foam and nonwoven layers laminated together.

In an infected wound, the exudate production is typically large. A wound dressing having the construction as explained hereinabove is suitable for handling large amounts of exudate and for preventing maceration of the skin surrounding the wound. Thus, the wound dressing is particularly suited for infection prevention.

The wound dressing 2 typically further comprises a release liner detachably attached to the adhesive skin-contact layer 19. The release liner may comprise one or a plurality of release liner portions and is configured to cover the entire surface of the adhesive skin-contact layer 19. In figure 2, one of the release liner portions is shown and is denoted 21.

As mentioned hereinbefore, the wound dressing 2 is preferably void of any sensors and of any electrical components.

According to another aspect, there is provided a kit comprising the first electronic circuit patch as described hereinbefore and at least one wound dressing.

The kit may further comprise at least one sensor strip, preferably a suture sensor strip.

The kit may also comprise a processing device, preferably wherein the processing device is comprised in a conformable pad, as described hereinbefore.

The kit may also comprise a wetting agent, preferably wherein the wetting agent is selected from water, saline, and an antiseptic solution.

The components of the kit may be provided in the same package.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. An electronic circuit patch (1) for use in conjunction with a wound dressing (2) and having a lateral (x) and a longitudinal (y) extension, wherein said patch (1) comprises:
- a transfer layer (3) having a top side (3a) and a bottom side (3b), wherein at least said bottom side (3b) is adhesive;
- a support layer (4) arranged on said top side (3a) of said transfer layer (3); and
- a release liner (5) releasably attached to said bottom side (3b) of said transfer layer (3);
**characterized in that** said patch (1) comprises an aperture (6) extending through at least said transfer layer (3) and **in that** said patch (1) comprises a first electronic circuit (7) arranged along at least a portion of said aperture (6).

2. The electronic circuit patch (1) according to claim 1, wherein said support layer (4) has a top side (4a) and a bottom side (4b), wherein said first electronic circuit (7) is arranged on said top side (3b) of said transfer layer (3) or on said bottom side (4b) of said support layer (4).

3. The electronic circuit patch (1) according to claim 1 or claim 2, wherein said support layer (4) is configured to be released from said top side (3a) of said transfer layer (3) upon wetting said support layer with a wetting agent, preferably wherein said wetting agent is selected from water, saline, and an antiseptic solution.

4. The electronic circuit patch (1) according to claim 2 or claim 3, wherein said electronic patch (1) further comprises a water-soluble coating arranged on said bottom side (4b) of said support layer (4).

5. The electronic circuit patch (1) according to any one of the preceding claims, wherein said transfer layer (3) has a thickness in the range of from 0.2 to 20 µm, preferably from 0.4 to 10 µm, more preferably from 1.0 to 6.0 µm.

6. The electronic circuit patch (1) according to any one of the preceding claims, wherein said patch (1) is divided into three separate portions along the lateral (x) extension of said patch (1): a first lateral edge portion (1a), a central portion (1b), and a second lateral edge portion (1c), wherein said aperture (6) is arranged in said central portion (1b) of said patch (1).

7. The electronic circuit patch (1) according to any one of the preceding claims, wherein said aperture (6) has a surface area of from 5 to 100 cm², preferably from 10 to 50 cm².

8. The electronic circuit patch (1) according to any one of the preceding claims wherein said aperture (6) is defined by a first lateral edge (6a), a second lateral edge (6b), a first longitudinal edge (6c) and a second longitudinal edge (6d); said first (6a) and second (6b) lateral edges extending in the longitudinal (y) direction of said patch (1); said first (6c) and second (6d) longitudinal (y) edges extending in the lateral (x) direction of said patch (1), and wherein said first electronic circuit (7) comprises a first branch (7a) extending adjacent to and along said first longitudinal edge (6c) of said aperture (6), and a second branch (7b) extending adjacent to and along said second longitudinal (y) edge (6d) of said aperture (6).

9. The electronic circuit patch (1) according to claim 8, wherein said first (7a) and second (7b) branches of said first electronic circuit (7) extend uninterrupted to an electronic connector (8) arranged in said second lateral edge portion (1c) of said patch (1).

10. A wound dressing assembly (9) comprising the electronic circuit patch (1) according to any one of claims 1-9, and a wound dressing (2), wherein said wound dressing (2) comprises a wound pad (10), and wherein said wound pad (10) is arranged on top of said aperture (6) of said patch (1) during use.

11. The wound dressing assembly (9) according to claim 10, wherein said wound dressing (2) is defined by peripheral edges, and wherein at least said electronic connector (8) is arranged in an area outside said peripheral edges of said wound dressing (2) during use.

12. The wound dressing assembly (9) according to claim 10 or claim 11, wherein said wound pad (10) is arranged in a central portion of said wound dressing (2), and wherein said wound dressing (2) further comprises an adhesive border portion (11) surrounding said central portion; said adhesive border portion (11) being arranged to extend beyond said first (6a) and second (6b) lateral edges and said first (6c) and second (6d) longitudinal edges of said aperture (6) during use.

13. The wound dressing assembly (9) according to any one of claim 10-12, further comprising at least one sensor strip (12), wherein said sensor strip (12) comprises a second electronic circuit (13) extending in a length direction of said sensor strip (12) and at least one sensor (14) connected to said second electronic circuit (13), preferably wherein said sensor strip (12) is a suture sensor strip.

14. The wound dressing assembly (9) according to any one of claims 10-13, further comprising a wetting agent, preferably wherein said wetting agent is an antiseptic solution.

15. The wound dressing assembly (9) according to any one of claims 10-14, further comprising a separate processing device configured for connection with said electronic connector (8), preferably wherein said processing device is arranged in a conformable pad (15).

16. The wound dressing assembly (9) according to any one of claims 10-15, wherein said wound dressing (2) is void of sensors and electronic circuits.
